# EUROPEAN PATENT SPECIFICATION

(11) **EP 2 524 664 B1**
(45) Date of publication and mention of the grant of the patent: **01.02.2017**
(21) Application number: 12168467.4
(22) Date of filing: 18.05.2012
(51) Int. Cl.: A61B 90/00, A61B 17/00

(54) **Integrated surgical visualization apparatus and systems**
Integrierte chirurgische Visualisierungsvorrichtungen und Systeme
Appareil et systèmes de visualisation chirurgicale intégrée

(30) Priority: 19.05.2011 US 201161487740 P; 12.04.2012 US 201213444908
(43) Date of publication of application: 21.11.2012
(73) Proprietor: Covidien LP, Mansfield, MA 02048 (US)
(72) Inventor: Stopek, Joshua, Guilford, CT Connecticut 06437 (US)
(74) Representative: Soames, Candida Jane

(56) References cited:
- WO-A1-2009/144729
- DE-U1-202006 011 532
- US-A1- 2005 014 994
- US-A1- 2005 143 624
- US-A1- 2006 149 135
- US-A1- 2008 167 546

## Description

### TECHNICAL FIELD

The present disclosure relates to an integrated surgical visualization apparatus, and system. More specifically, the present disclosure relates to the integration of small cameras and visualization technologies into fixation and/or stabilization surgical devices for providing improved visualization during surgery.

### BACKGROUND OF RELATED ART

Endoscopic and laparoscopic minimally invasive procedures have been used for introducing medical devices inside a patient and for viewing portions of the patient's anatomy. Typically, to view a desired anatomical site, a surgeon may insert a rigid or flexible endoscope inside the patient to render images of the anatomical site. In laparoscopic or minimally invasive procedures, visualization technologies are typically centered on the direct integration of the camera component onto the end effector of a surgical instrument, i.e. grasper, clip applier, stapler, and the like, for observing "line of sight" and immediate space in which the respective instrument interrogates tissue. Because the camera is part of the surgical instrument, during a procedure, the surgeon is required to bring the tip of the instrument close to the surgical site, thus eliminating a global view of the surgical site.

The direct integration of the camera component onto the surgical instrument may compromise the ability of the surgeon to manipulate between the camera and the surgical tools when all devices are located along a single axis. In addition, introducing the camera and the surgical tools through working channels of the instrument may compromise its flexibility. Furthermore, during surgical procedures, the surgeon often navigates the instruments through tortuous paths and, thus, the rotational orientation of the instrument may not be aligned with the expected surgical view of the anatomical site. Moreover, the presence of the camera and associated wiring within the instrument takes up space and may interfere with the procedure.

Accordingly, there is a need for an effective, hands free integrated camera/ visualization approach which provides a global view of the surgical site.

DE202006011532U describes a surgical instrument containing small devices in a longitudinal shaft thereof. The small devices may be cameras, optionally including light sources.

US2005/0014994A describes implantable imaging devices for use in endoscopic surgery.

US2006/0149135A describes devices auxiliary to surgery, for anchoring and lifting cavity walls or internal organs of a patient. The device provides a virtual port, that is an instrument that can be non-invasively, or minimally invasively and removably attached to the undersurface of a patient's cavity, or to various tissues within a cavity, and to which various retracting means are attached.

WO2009/144729A describes multi-camera arrays for laparoscopic use, mounted on rigid intra-body structures, and deployed in predefined fixed positions and orientations.

US2008/0167546A describes apparatus for imaging the anatomy including securing a scanner assembly to an anatomical structure, and scanning the anatomy with the scanner assembly secured to the anatomical structure.

US2005/0143624A describes an in-vivo imaging device that can be secured to an in-vivo surface.

### SUMMARY

In a first aspect, the present invention provides a surgical visualization apparatus, comprising: a fixation device having an upper surface and a tissue penetrating surface; an imaging device housed within the fixation device, the imaging device comprising a camera including at least one lens directed outwardly from the upper surface of the fixation device such that when the fixation device is inserted in tissue, the at least one lens is oriented away from the tissue; wherein the fixation device is bioabsorbable.

In a second aspect, the present invention provides a integrated fixation visualization system, comprising a surgical visualization apparatus according to the present invention, and further comprising: a delivery device including a delivery tube with a proximal and distal end, and an actuator; with a plurality of fixation devices disposed within the delivery device; wherein: the delivery device includes a housing and a handle disposed on the proximal end; and the integrated fixation visualization system further comprises a monitor for displaying images.

The apparatus and systems of the present invention may be used in a method of delivering an integrated fixation visualization apparatus. The method includes providing a delivery device having a plurality of fixation devices disposed therein, each fixation device having an upper surface and a tissue penetrating surface, wherein at least one of the plurality of fixation devices includes at least one camera coupled thereto, the at least one camera including at least one lens directed outwardly from the upper surface of the fixation device; deploying the at least one of the plurality of fixation devices in a predetermined location in patient's tissue such that the at least one lens is oriented away from the patient's tissue surface for capturing images of an internal cavity of the patient.

In embodiments, the illumination source of each fixation device may be of different wavelengths or types of light. Such light may activate other devices or therapies, such as UV light to activate polymerization.

In further embodiments, the each visualization device may be arrayed on a common carrier such as a surgical mesh. The mesh may be self adhesive to tissue or retained by various fastening systems which are preferably formed of resorbable polymers so that the visualization array may be removed at the conclusion of surgery. Such an array may also incorporate wires to transmit power and signals to and from the cameras, illuminators or transmitters of the visualization devices.

### BRIEF DESCRIPTION OF THE DRAWINGS

The above and other aspects, features, and advantages of the present disclosure will become more apparent in light of the following detailed description when taken in conjunction with the accompanying drawings in which:
FIG. 1A and FIG. 1B are side perspective views of an integrated fixation visualization apparatus in accordance with the embodiments of the present disclosure;
FIG. 2A and FIG. 2B are side cross sectional views of the integrated fixation visualization apparatus as shown in FIG. 1a and 1b, respectively;
FIG. 3 is a side view of an integrated fixation visualization apparatus delivery device in accordance with an embodiment of the present disclosure;
FIG. 4 is a side cross sectional view of the proximal portion of the delivery device of FIG. 3;
FIG. 5 is a side cross sectional view of the distal portion of the delivery device of FIG. 3;
FIG. 6 is an enlarged view of the area detail of FIG. 5;
FIG. 7 depicts the delivery device in a deployed position proximate patient's tissue in accordance with an embodiment of the present disclosure;
FIG. 7A depicts the delivery device in a deployed position proximate patient's tissue in accordance with an alternate embodiment of the present disclosure;
FIG. 8 shows the integrated fixation visualization apparatus after penetration of the tissue and before release of the trigger in accordance with an embodiment of the present disclosure;
FIG. 9 shows the deployed integrated fixation visualization apparatus in patient's tissue after ejection from the delivery device in accordance with an embodiment of the present disclosure;
FIG. 10 depicts a plurality of integrated fixation visualization apparatus in patient's tissue in accordance with an embodiment of the present disclosure; and
FIG. 11 illustrates an integrated fixation visualization system in accordance with an embodiment of the present disclosure.

### DETAILED DESCRIPTION

Particular embodiments of the present disclosure are described hereinbelow with reference to the accompanying drawings; however, it is to be understood that the disclosed embodiments are merely exemplary of the disclosure and may be embodied in various forms. Well-known functions or constructions are not described in detail to avoid obscuring the present disclosure in unnecessary detail. Therefore, specific structural and functional details disclosed herein are not to be interpreted as limiting, but merely as a basis for the claims and as a representative basis for teaching one skilled in the art to variously employ the present disclosure in virtually any appropriately detailed structure. Like reference numerals may refer to similar or identical elements throughout the description of the figures.

As used herein, the term "distal" refers to that portion of the instrument, or component thereof which is farther from the user while the term "proximal" refers to that portion of the instrument or component thereof which is closer to the user.

Various embodiments of integrated fixation visualization device, system and methods disclosed herein may be employed in endoscopic, laparoscopic, open surgical procedures, interventional and/or intralumenal procedures such as GI sheathing (metabolic/bariatric) and/or banding, and/or for more advanced minimally invasive procedures such as those which employ a device that facilitates multiple instrument access through a single incision and permits a user to operate through a single entry point, i.e., navel, vagina and/or anus, and combinations thereof, where additional visualization due to compromising space, is required. In addition, the system of the present disclosure may be utilized for post-operative monitoring, diagnostics and combinations thereof.

In embodiments, the integrated fixation visualization apparatus, system and methods of the present disclosure may be utilized in lieu of or adjunctive to a traditional scope and/or surgical instrument, and the apparatus may be specifically designed for use with instruments including an endoscope and additional instruments such as graspers, staplers, forceps or the like introduced within a portal member to carry out the surgical procedure, and/or other access devices. An example of such a surgical portal is disclosed in commonly assigned U.S. Patent Application Publication No. 2009/0093752 A1, filed October 2, 2008.

In embodiments, the device may be used to guide other instruments by sight or electronically to very precise anatomical sites, such as for example tumor and/or disease sites. In embodiments, for example, the apparatus may be utilized for complex thoracic surgeries where the apparatus may be deployed into the chest wall or lung directly for added visualization of critical vessels and/or pulmonary structures. In other embodiments, the integrated fixation visualization apparatus may be used to communicate with visualization technology utilized in a surgical instrument, i.e. a laparoscopic instrument, for positioning of the surgical instrument to the target or predetermined anatomical site.

Various embodiments of the integrated fixation visualization apparatus of the present disclosure may comprise devices inserted in a patient to provide visualization of the target site. These devices may be introduced into the patient using minimally invasive procedures through natural orifices such as those mentioned above, or via a device inserted through a trocar, for example, and may be adapted to provide images of the surgical site or anatomic location such as the lungs, liver, stomach, gall bladder, urinary tract, reproductive tract, and intestinal tissue, for example. Once positioned at the target site, the integrated fixation visualization devices provide images that enable the surgeon to more accurately diagnose and provide more effective treatment of the diseased tissue. In embodiments, the integrated fixation visualization apparatus may be inserted into the tissue treatment region percutaneously. In other embodiments, the integrated fixation visualization device may be introduced into the tissue treatment region endoscopically (e.g., laparoscopically and/or thoracoscopically), through small keyhole incisions via a trocar, or through a natural orifice.

Embodiments of the integrated fixation visualization devices may provide images of the desired tissue during in-vivo treatment procedures used to ablate or destroy live cancerous tissue, tumors, masses, lesions, and other abnormal tissue growths present at the tissue treatment site. In embodiments, the integrated fixation visualization devices may be configured to transmit electrical signals to a receiver and then convert the signals into a viewable image. The signals may be transmitted outside the patient either wirelessly or through electrical conductors placed percutaneously or through the same access path as the translumenal endoscopic access device. Other embodiments of the integrated fixation visualization devices may be powered by on-board power sources, such as a battery, percutaneous electrical conductors, wireless power conductors, or electrical conductors introduced along the same path as the translumenal endoscopic access devices.

In various other embodiments, a variety of integrated fixation visualization end effector devices may be coupled to a suitable applier and introduced through the flexible working channel of an endoscope introduced inside a patient through a natural opening. Suitable examples of such integrated fixation visualization end-effectors include, but are not limited to graspers, clip appliers, staplers, retraction clips, tissue clamps, endoscope stabilizers, electrical power distribution devices, space creators such as devices configured to create space between internal body lumen, organs, and/or dissected sections of tissue, pace makers, vascular access ports, injection ports (such as used with gastric bands), and gastric pacing devices, among other devices.

### The Integrated Fixation Visualization Apparatus

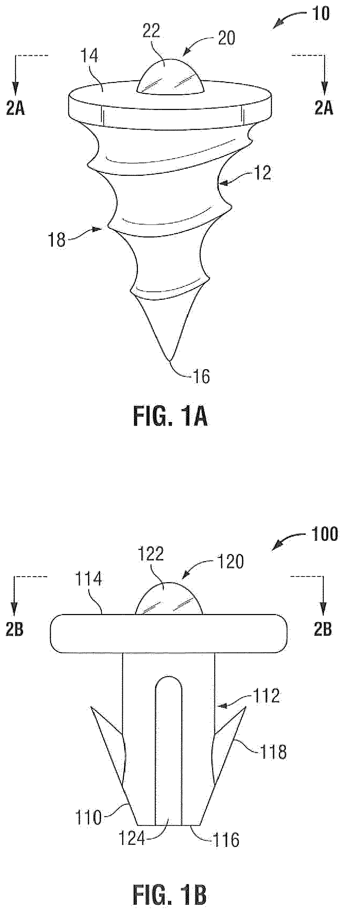

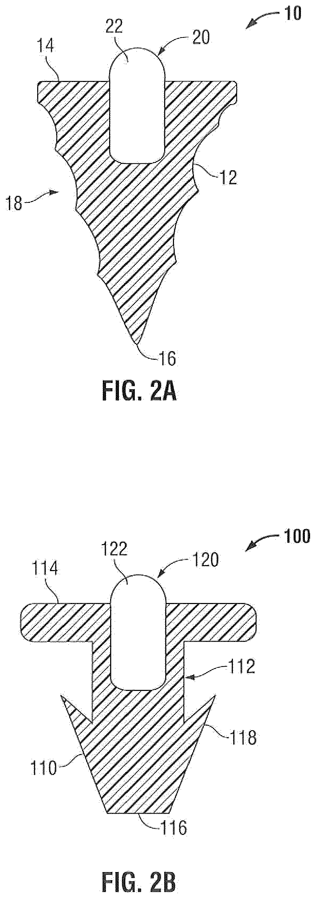

FIGS. 1A and 2A illustrate an embodiment of an integrated fixation visualization apparatus 10. The integrated fixation visualization apparatus 10 includes a fixation device 12 having an upper surface 14 and a distal end or tissue penetrating surface 16. The fixation device 12 further includes a threaded tissue penetrating section 18 which spiral in either a right hand or left hand manner from the distal end of the upper surface to the distal end of the tissue penetrating surface 16. Threaded section 18 engages tissue and holds integrated fixation apparatus 10 in position.

The integrated fixation visualization apparatus 10 further includes an imaging device 20 housed within the fixation device 12. The imaging device 20 includes at least one camera 22 having at least one lens (not shown) directed outwardly from the upper surface 14 of the fixation device 12 such that when the fixation device 12 is inserted in tissue, the at least one lens is oriented away from tissue.

FIGS. 1B and 2B illustrate an alternative embodiment of an integrated fixation visualization apparatus 100. The integrated fixation visualization apparatus 100 includes a fixation device 112 having an upper surface 114 and a tissue penetrating surface 116. The fixation device 112 further includes barbs 118 which may be rigid and formed integral to the penetration shaft 110 of fixation device 112. Penetration shaft 110 is tapered from the distal most point on barbs 118 to the blunt distal end of the fixation device 112. Slits 124 in distal end of the penetration shaft 110 allow distal end to flex to facilitate ejection of the fixation device 112 from a delivery device, which is described in more detail below.

The integrated fixation visualization apparatus 110 further includes an imaging device 120 housed within the fixation device 112. The imaging device 120 includes at least one camera 122 which includes at least one lens (not shown) directed outwardly from the upper surface 114 of the fixation device 112 such that when the fixation device 112 is inserted in tissue, the at least one lens is oriented away from tissue.

In embodiments, the upper surface 14, 114 diameter of the fixation device 12, 112 may be from about 4 mm to about 8 mm, in embodiments from about 5 mm to about 7 mm. In embodiments, the threaded tissue penetrating section 18 or penetration shaft 110 diameter of the fixation device 12, 112 may be from about 2 to about 5 mm, in embodiments about 3 mm. In embodiments, the overall length of the fixation device 12, 112 may be from about 4 mm to about 8 mm, in embodiments from about 5 mm to about 7 mm. In embodiments, the diameter of the imaging device 20, 120 may be from about 2 to about 4 mm, in embodiments about 3 mm.

In embodiments, the imaging device 20, 120 may include one or more light or illumination sources (not shown). In other embodiments, the integrated fixation visualization apparatus may also include one or more light or illumination source to illuminate the site to be imaged. In embodiments, illumination may be achieved using a solid state element, such as a light emitting diode (LED) based light source of various wavelength, including those for surgical illumination and/or for detecting disease. In embodiments, the light source may include a single LED or combination LED to provide light of the appropriate spectrum. In embodiments, the LED based light source may be white or fluorescent. Alternative illumination sources may include fluorescent and/or near infrared light with an optical and/or digital filter. In other embodiments, fiber optic light sources may be introduced through the working channel of a flexible endoscope.

In embodiments, the integrated fixation visualization apparatus 10, 100 may be configured to work synergistically with dyes and/or probes that illuminate specific tissue structures such as nerves, vessels, and ureters, organs, diseases such as tumors, chronic inflammatory disease, etc. and/or injuries.

In embodiments, the imaging device 20, 120 may be rotated about its optical axis, translated forward and rearward, and may be rotated about a pivot point defined by a tissue keyhole site to control its orientation and obtain a quality image at a desired viewing angle. In embodiments, the imaging device 20, 120 may be gyrated within the fixation device 12, 112 to provide preferred views. In addition, the imaging device 20, 120 may be used to pan and/or zoom the images while the surgeon manipulates other surgical instruments to the surgical site.

In embodiments, the lens may be optically coupled to one or more image sensors (not shown) to convert an optical image to an electric signal, similar to that employed in digital cameras and other electronic imaging devices.

In one embodiment, the image sensor may include one or more arrays of charge coupled devices (CCD), charge injection devices (CID) and/or complementary metal oxide semiconductor (CMOS) devices such as active-pixel sensors. The image sensor may capture light and convert it into electrical signals. In one embodiment, the image sensor may include a sensor array with an image input area of approximately 2 mm diameter.

In embodiments, the image sensor may be connected to a circuit board (not shown) including any necessary electronic components or elements for processing, storing, and/or transmitting the images received by the image sensor. The images may be processed by any suitable digital or analog signal processing circuits and/or techniques. Furthermore, the images may be stored in electronic storage media such as, for example, memory devices. In embodiments, the images may be transmitted over a wire or wirelessly to external devices for displaying or further processing the images in real-time. A second circuit board (not shown) may be employed to receive and attach a battery and is coupled to the first circuit board by a connector (not shown).

In embodiments, the imaging device 20, 120 may include a wireless component for wirelessly transmitting images outside the patient and may include a battery (not shown) to operate various electrical and/or electromechanical elements of the camera 22, 122. For example, the battery may supply electrical energy to power light sources, image sensor arrays, and motors for orienting, panning, and zooming the image sensor arrays or the associated optics or lenses. In embodiments, the wireless component may be a radio frequency (RF) device suitable for transmitting images remotely from the patient to an external monitor. The wireless component may be powered either by a battery or by a power source through electrical conductors. In one embodiment, the wireless component may include a wireless transceiver (e.g., RF transmitter and receiver) module. Images received by the image sensor may be wirelessly transmitted/received between the wireless RF device using any well known RF telemetry techniques so as to eliminate the need for hard wired electrical connections.

In embodiments, a single or plurality of integrated visualization apparatus 10, 100 may be deployed into tissue or anatomical location such that images and/or videos from each camera may be stitched together to provide a global view of the surgical field. In embodiments, the images provided of the surgical site may be 2-dimensional, 3-dimensional, wide angle, and combinations thereof.

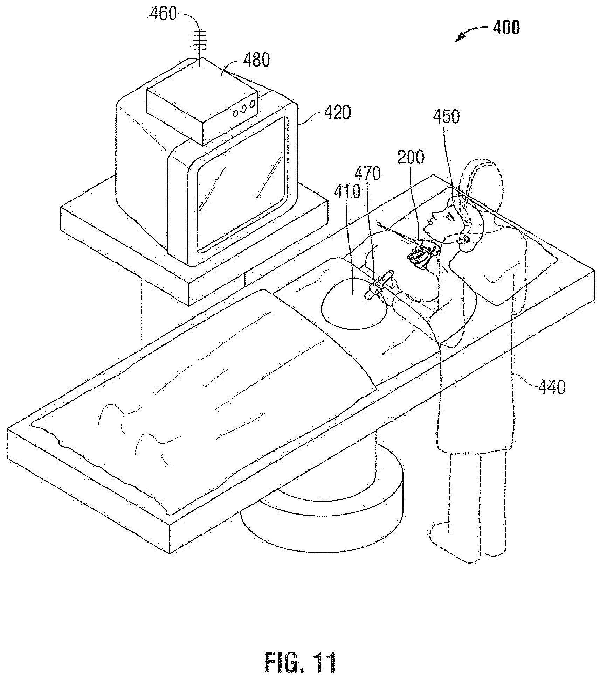

In embodiments, the images and/or videos are viewable on a display or monitor 420 as illustrated in the schematic drawing of an integrated fixation visualization system of FIG. 11. As illustrated, surgeon 440 has deployed the integrated visualization apparatus 10 into patient's 450 surgical site 410 with a delivery device 200 described in detail below. The surgeon 440 may then perform the predetermined procedure with the appropriate surgical instrument 470.

In embodiments, an optical image of the surgical site 410 may be formed on the image sensors through the optical lens system of the camera 20, 120. Image signals into which image sensors (i.e., a CCD image sensor) convert the optical image formed thereon are processed in an image signal processor (not shown) and then sent to the image signal processing unit 480 from a transmitter (not shown) through an antenna 460.

The fixation device 12, 112 may be any device suitable of anchoring to tissue such as tacks, darts, barbed implants, gripped implants, fasteners and combinations thereof.

The fixation device 12, 112 described herein may be formed from any biocompatible natural or synthetic, bioabsorbable material. The term "bioabsorbable" as used herein is defined to include both biodegradable and bioresorbable materials. By bioabsorbable, it is meant that the materials decompose, or lose structural integrity under body conditions (e.g. enzymatic degradation or hydrolysis) or are broken down (physically or chemically) under physiologic conditions in the body such that the degradation products are excretable or absorbable by the body.

Representative natural bioabsorbable materials include: polysaccharides, such as alginate, dextran, chitin, hyaluronic acid, cellulose, collagen, gelatin, fucans, glycosaminoglycans, and chemical derivatives thereof (substitutions and/or additions of chemical groups, for example, alkyl, alkylene, hydroxylations, oxidations, and other modifications routinely made by those skilled in the art); and proteins, such as albumin, casein, zein, silk, and copolymers and blends thereof, alone or in combination with synthetic polymers.

Synthetically modified natural polymers include cellulose derivatives, such as alkyl celluloses, hydroxyalkyl celluloses, cellulose ethers, cellulose esters, nitrocelluloses, and chitosan. Examples of suitable cellulose derivatives include methyl cellulose, ethyl cellulose, hydroxypropyl cellulose, hydroxypropyl methyl cellulose, hydroxybutyl methyl cellulose, cellulose acetate, cellulose propionate, cellulose acetate butyrate, cellulose acetate phthalate, carboxymethyl cellulose, cellulose triacetate, and cellulose sulfate sodium salt. These are collectively referred to herein as "celluloses."

Representative synthetic bioabsorbable polymers include polyhydroxy acids prepared from lactone monomers, such as glycolide, lactide, caprolactone, ε-caprolactone, valerolactone, and δ-valerolactone, as well as pluronics, carbonates (e.g., trimethylene carbonate, tetramethylene carbonate, and the like), dioxanones (e.g., 1,4-dioxanone and p-dioxanone), l,dioxepanones (e.g., 1,4-dioxepan-2-one and 1,5-dioxepan-2-one), and combinations thereof. Polymers formed therefrom include: polylactides; poly(lactic acid); polyglycolides; poly(glycolic acid); poly(trimethylene carbonate); poly(dioxanone); poly(hydroxybutyric acid); poly(hydroxyvaleric acid); poly(lactide-co-(ε-caprolactone-)); poly(glycolide-co-(ε-caprolactone)); polycarbonates; poly(pseudo amino acids); poly(amino acids); poly(hydroxyalkanoate)s, including polyhydroxybutyrate, polyhydroxyvalerate, poly(3-hyydroxybutyrate-co-3-hydroxyvalerate), polyhydroxyoctanoate, and polyhydroxyhexanoate; polyalkylene oxalates; polyoxaesters; polyanhydrides; polyortho esters; and copolymers, block copolymers, homopolymers, blends, and combinations thereof.

### The Delivery Device

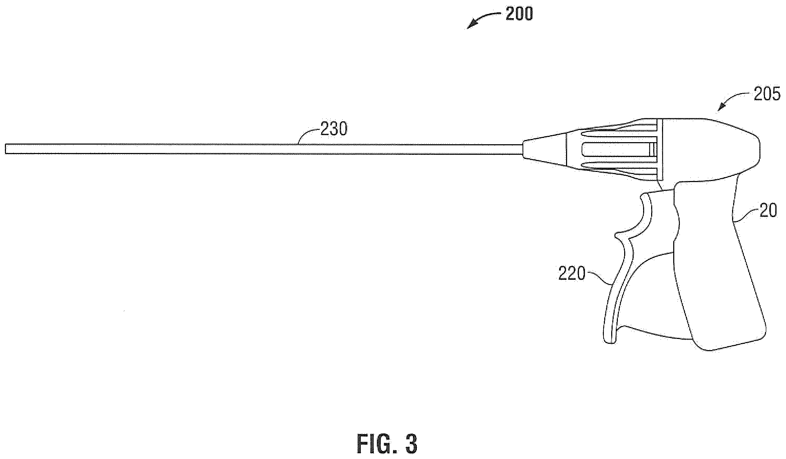

In embodiments, any suitable delivery device may be utilized to deploy the integrated fixation visualization apparatus 10, 100 of the present disclosure into patient tissue at a target surgical site, such as for example, the delivery device disclosed in U.S. Patent Application Publication No. 2010/0312258. The delivery device can be best understood by referring to FIGS. 3-6. FIG. 3 depicts a side view of the delivery device, generally designated with the number 200. Delivery device 200 comprises a housing 205, pistol grip handle 210, an actuator or trigger 220 and delivery tube 230. In embodiments, the outside diameter of delivery tube 230 is approximately 5 mm for use with standard trocars, laparoscopic devices for minimally invasive entry into the abdomen. In embodiments, handle 210 and trigger 220 may be formed from plastic material such as ABS or polycarbonate but alternately may be formed from metal to facilitate reuse and resterilization. In embodiments, delivery tube 230 may be formed from thin wall stainless steel but can alternately be formed from rigid biocompatible plastic material.

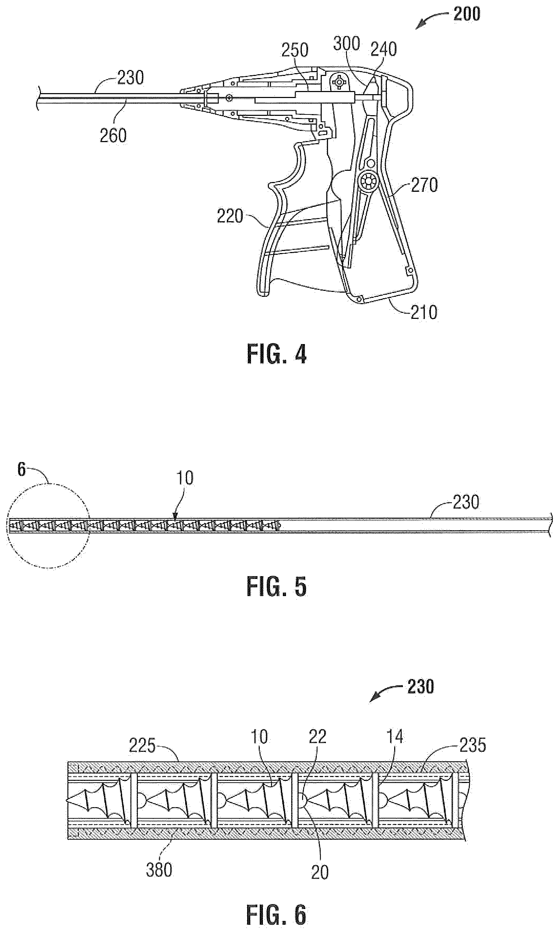

As can be seen in FIGS. 4-6 delivery tube 230 contains a plurality of integrated fixation visualization devices 10. In embodiments, delivery tube 230 may be designed so that it is readily detachable from housing 205 thus resulting in a reusable handle and a reloadable or replaceable tube, otherwise the entire delivery device is for use only in a single surgical procedure.

FIG. 4 is a longitudinal cross section of the proximal and distal ends respectively of delivery device 200 in the home or equilibrium state. Trigger 220 abuts rotating lever 240 which is spring loaded with torsion spring 270. Anchor carrier 260, connected proximally to piston 250, comprises a cylindrical rod terminating distally in integrated fixation visualization apparatus 10. Anchors carrier 260 may be fed inside internal channels (not shown) of the integrated fixation visualization devices 10 such that integrated fixation visualization devices 10 are in upper surface 14-to tissue penetrating surface 16-contact such that tissue penetrating surface 16 of integrated fixation visualization apparatus 10 are aligned toward distal end of delivery device 200. The proximal end of queuing spring 280 is fixed with respect to delivery tube 230 and the distal end of queuing spring 280 abuts upper surface 14 of proximal-most integrated fixation visualization apparatus 10. Queuing spring 280 is compressed and serves to urge integrated fixation visualization apparatus 10 distally against each other and against the proximal shoulder of integrated fixation visualization apparatus 10 that abuts tissue penetrating surface 16 of distal-most integrated fixation visualization apparatus 10 and provides a counter force against queuing spring 280. Tissue penetrating surface 16 of integrated fixation visualization apparatus 10 and the proximal end of integrated fixation visualization apparatus 10 are each formed such that there is a smooth transition between the two components. This minimizes the insertion force required to set integrated fixation visualization apparatus 10 into the tissue.

FIG. 5 depicts the distal end of delivery device 200 with a plurality of integrated fixation visualization devices 10 loaded, ready for use. In embodiments, the delivery tube 230 may include an outer tube 225 and an inner tube 235.

FIG. 6 is cutaway view of an enlargement of the distal end of delivery device 200 depicting the distal most integrated fixation visualization devices 10. In embodiments, Upper surface 14 of integrated fixation visualization devices 10 engage internal screw threads 380 in delivery tube 230. The distal end of inner tube 235 is slotted to accept multiple integrated fixation visualization devices 10. In embodiments, rotation of inner tube 235 about its longitudinal axis rotates integrated fixation visualization devices 10 may advance them distally owing to the upper surface 14 engagement with outer tube 225 by threads 380. In embodiments, integrated fixation visualization devices 10 are not in forced engagement with each other to avoid damage to tissue penetrating surface 16 of integrated fixation visualization devices 10.

FIGS. 5 and 6 depict delivery device 200 in the fully stroked state. In this fully stroked state trigger 220 is locked to handle 210 by the surgeon's closed hand and no further activation of delivery device 200 is necessary to set the integrated fixation visualization device 10 into tissue. With respect to the alternative embodiment of integrated fixation visualization device 100, barbs 14 lock distal-most integrated fixation visualization device 100 into the tissue.

In use, when the surgeon pulls trigger 240 proximally, away from the home state, lever 240 rotates counterclockwise such that cam surface 300 of lever 240 contacts piston 250 which drives integrated fixation visualization device carrier rod 260 distally. Torsion spring 270 compresses as lever 240 is rotated counterclockwise. Integrated fixation visualization device carrier rod 260 is urged distally within the inside diameter of queuing spring 280.

### The Method

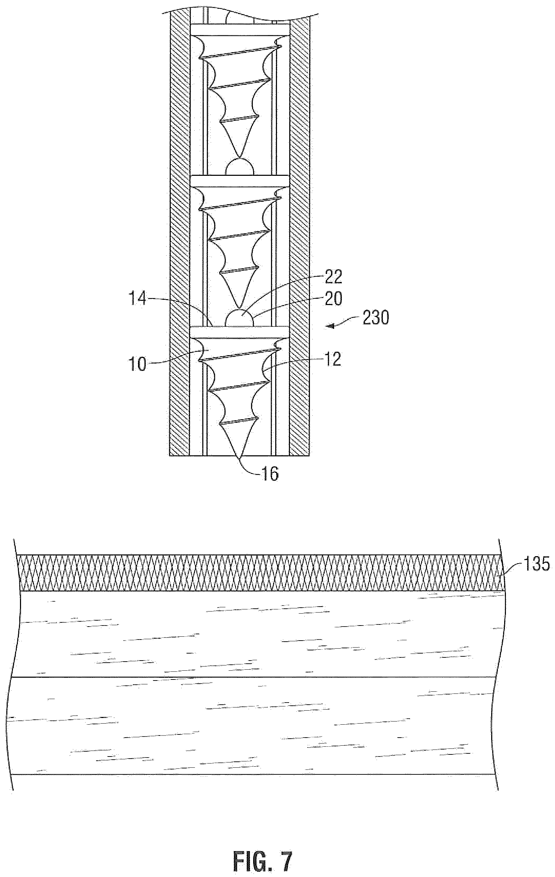

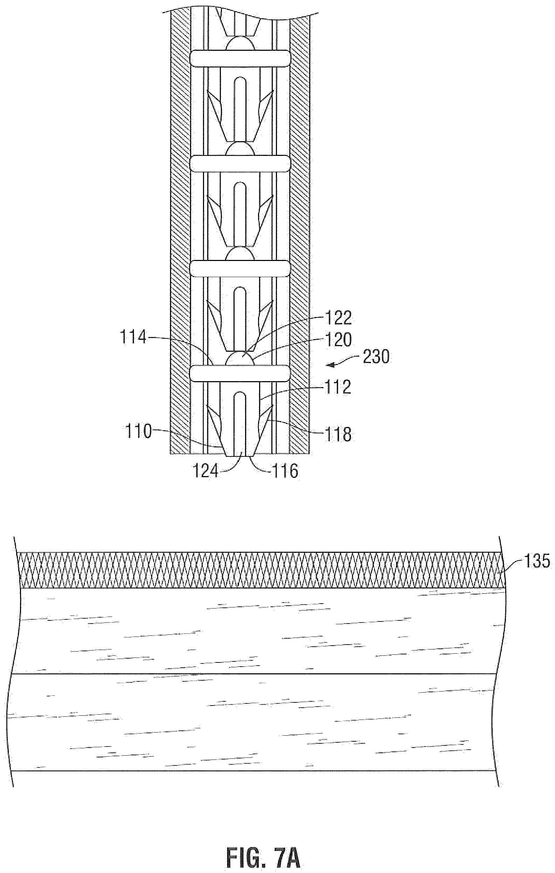

In embodiments, FIGS. 7-9 illustrate the method of delivering an integrated fixation visualization apparatus 10. These longitudinal cross-sectional views of the distal end of delivery tube 230 show the steps involved in using delivery device 200 and integrated fixation visualization apparatus 10 for deploying and securing at least one fixation device 12 in a predetermined location in patient's tissue. In embodiments, the method may include illuminating the target area with a light source as described in detail above.

FIG. 7 depicts the distal end of the delivery device 200 proximate tissue 135. FIG. 7A depicts the distal end of the delivery device 200 proximate tissue utilizing the alternative embodiment of integrated visualization apparatus 100 with barbs 118.

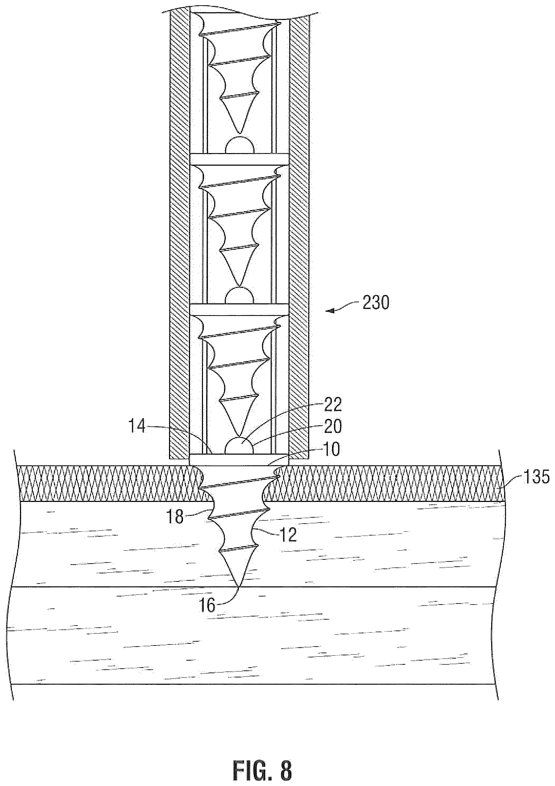

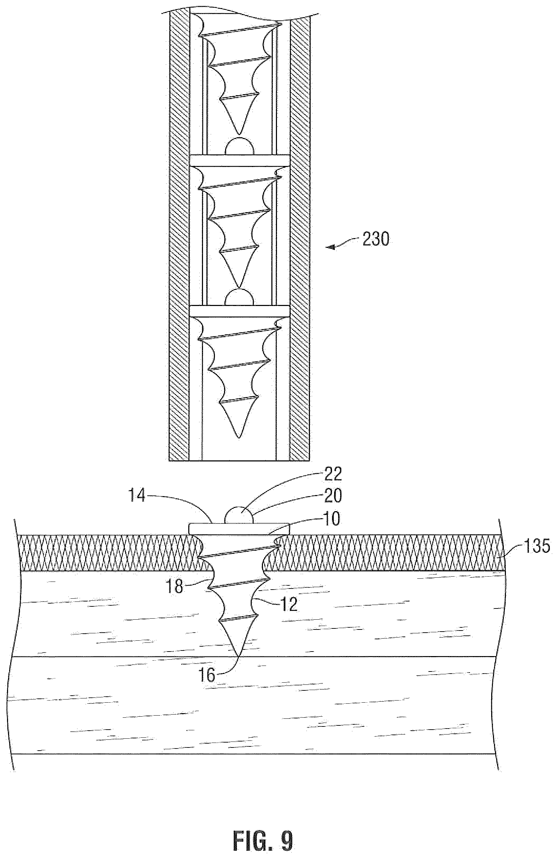

In FIG. 8, the distal end of delivery device 200 is shown deploying integration fixation visualization apparatus 10 and fully penetrating tissue. The surgeon pulls trigger 220 fully proximal exposing tissue penetrating surface 16 and tissue penetrating section 18 distally from delivery tube 230. The surgeon then urges the entire assembly forward so that tissue penetrating surface 16 and tissue penetrating section 18 have penetrated tissue 135. The surgeon moves delivery device 200 proximally and withdraws delivery device 200 from contact with tissue 135. In embodiments, the threaded tissue penetrating section 18 may provide a counter force in the tissue so that integrated fixation visualization apparatus 10 remains in the tissue. In other embodiments, barbs 118 may provide a counter force in the tissue so that integrated fixation visualization apparatus 100 remains in the tissue. The surgeon then releases trigger 220. As illustrated in FIG. 9, the delivery device 200 is reset to the home position and is ready for deploying another integrated fixation visualization apparatus 10.

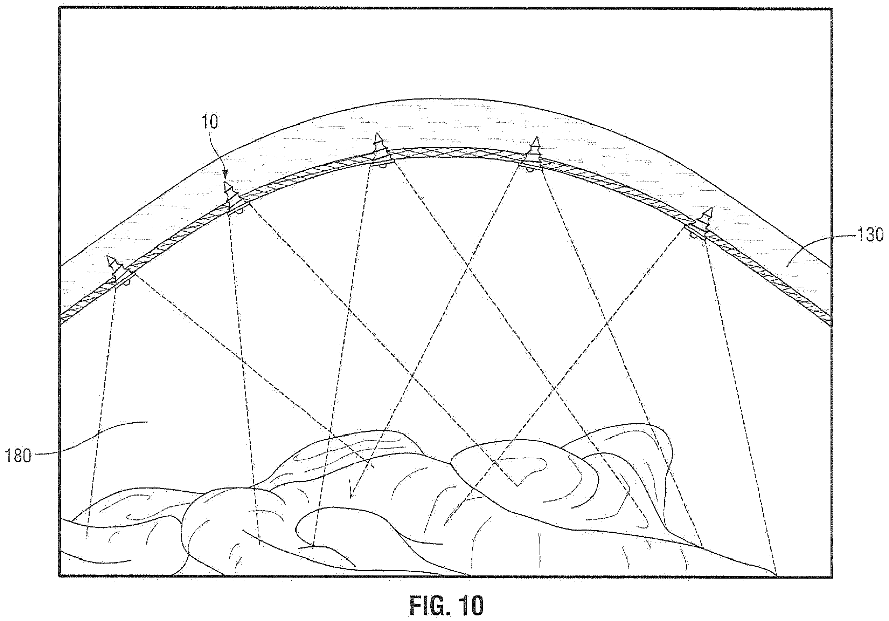

In embodiments, a single or series of integrated fixation visualization devices may be deployed in any tissue or anatomical location where the delivery device can access. As shown for example in FIG. 10, a plurality of integrated fixation visualization devices 10 may be deployed in predetermined spaced apart locations of patient tissue such that a field of view 180 of each adjacent camera overlaps a field of view 180 of the adjacent camera. In embodiments, images and/or videos from each camera may be processed or "stitched" together to provide a global view of the surgical field and/or a single image on a display.

In embodiments, once deployed into patient's tissue, the integrated fixation visualization apparatus 10, 100 may help guide and position a surgical instrument to a predetermined anatomical site via camera to camera communication either with the camera on the surgical instrument or with an adjacent integrated fixation visualization apparatus 10, 100.

In embodiments, the integrated fixation visualization apparatus 10, 100 may be configured and dimensioned such that the imaging device 20, 120 is removable following use utilizing the same deployment methods or any alternative secondary retrieval instrument. In embodiments, the imaging device 20, 120 may be removed by any means known in the art such as mechanical, magnetic, and/or chemical means.

While various embodiments of the present disclosure have been shown and described herein, it will be obvious to those skilled in the art that these embodiments are provided by way of example only. Numerous variations, changes, and substitutions will now occur to those skilled in the art without departing from the scope of the appended claims.

## Claims

1. A surgical visualization apparatus (10; 100), comprising:
a fixation device (12; 112) having an upper surface (14; 114) and a tissue penetrating surface (16; 116);
an imaging device (20,120) housed within the fixation device (12; 112), the imaging device comprising a camera (22; 122) including at least one lens directed outwardly from the upper surface (14; 114) of the fixation device (12; 112) such that when the fixation device (12; 112) is inserted in tissue, the at least one lens is oriented away from the tissue;
wherein the fixation device (12; 112) is bioabsorbable.

2. The surgical visualization apparatus (10; 100) according to claim 1, further comprising a light source.

3. The surgical visualization apparatus (10; 100) according to claim 2, wherein the light source includes light emitting diodes.

4. The surgical visualization apparatus (10; 100) according to any preceding claim, further comprising an optical filter.

5. The surgical visualization apparatus (10; 100) according to any preceding claim, wherein the fixation device (12; 112) is selected from the group consisting of tacks, darts, anchors, barbed implants, gripped implants, fasteners and combinations thereof.

6. The surgical visualization apparatus (10; 100) according to any preceding claim, wherein at least a portion of the camera (22; 122) is absorbable.

7. The surgical visualization apparatus (10; 100) according to any preceding claim, wherein the camera (22; 122) includes a material selected from the group consisting of glasses, ceramics, polymers, metals, and combinations thereof.

8. The surgical visualization apparatus (10; 100) according to any preceding claim, wherein the camera (22; 122) is wireless and includes devices selected from the group consisting of fiber optics, CMOS, CCD, CID, NIR, Raman, fluorescence based components, digital based components, optical based components, and variations thereof.

9. The surgical visualization apparatus (10; 100) according to any preceding claim, wherein the at least one lens is rotatable within the fixation device (12; 112) for capturing images of the tissue to provide preferred fields of view.

10. An integrated fixation visualization system (400), comprising an apparatus as claimed in any preceding claim, and further comprising:
a delivery device (200) including a delivery tube (230) with a proximal and distal end, and an actuator (220);
with a plurality of fixation devices (12; 112) disposed within the delivery device (200); wherein:
the delivery device (200) includes a housing (205) and a handle (210) disposed on the proximal end; and
the integrated fixation visualization system (400) further comprises a monitor (420) for displaying images.

## Patentansprüche

1. Eine chirurgische Visualisierungsvorrichtung (10; 100), aufweisend:
eine Verankerungsvorrichtung (12; 112) mit einer oberen Oberfläche (14; 114) und einer gewebedurchdringenden Oberfläche (16; 116);
eine Bildgebungsvorrichtung (20, 120), die innerhalb der Verankerungsvorrichtung (12; 112) beherbergt ist, wobei die Bildgebungsvorrichtung eine Kamera (22; 122) aufweist, die wenigstens eine Linse umfasst, die von der oberen Oberfläche (14; 114) von der Verankerungsvorrichtung (12; 112) nach außen gerichtet ist, so dass die wenigstens eine Linse weg von dem Gewebe orientiert ist, wenn die Verankerungsvorrichtung (12; 112) in Gewebe eingefügt wird;
wobei die Verankerungsvorrichtung (12; 112) bioresorbierbar ist.

2. Die chirurgische Visualisierungsvorrichtung (10; 100) nach Anspruch 1, weiter eine Lichtquelle aufweisend.

3. Die chirurgische Visualisierungsvorrichtung (10; 100) nach Anspruch 2, wobei die Lichtquelle lichtemittierende Dioden umfasst.

4. Die chirurgische Visualisierungsvorrichtung (10; 100) nach einem vorangegangenen Anspruch, weiter einen optischen Filter aufweisend.

5. Die chirurgische Visualisierungsvorrichtung (10; 100) nach einem vorangegangenen Anspruch, wobei die Verankerungsvorrichtung (12; 112) ausgewählt ist aus der Gruppe bestehend aus Zwecken, Pfeilen, Ankern, Implantaten mit Widerhaken, griffigen Implantaten, Befestigungsmitteln und Kombinationen davon.

6. Die chirurgische Visualisierungsvorrichtung (10; 100) nach einem vorangegangenen Anspruch, wobei wenigstens ein Abschnitt von der Kamera (22; 122) absorbierbar ist.

7. Die chirurgische Visualisierungsvorrichtung (10; 100) nach einem vorangegangenen Anspruch, wobei die Kamera (22; 122) ein Material ausgewählt aus der Gruppe bestehend aus Gläsern, Keramiken, Polymeren, Metallen und Kombinationen davon umfasst.

8. Die chirurgische Visualisierungsvorrichtung (10; 100) nach einem vorangegangenen Anspruch, wobei die Kamera (22; 122) drahtlos ist und Vorrichtungen ausgewählt aus der Gruppe bestehend aus Faseroptik, CMOS, CCD, CID, NIR, Raman, fluoreszenzbasierte Komponenten, digitalbasierte Komponenten, optisch basierte Komponenten und Variationen davon umfasst.

9. Die chirurgische Visualisierungsvorrichtung (10; 100) nach einem vorangegangenen Anspruch, wobei die wenigstens eine Linse innerhalb der Verankerungsvorrichtung (12; 112) drehbar ist, um Bilder von dem Gewebe aufzunehmen, um bevorzugte Sichtfelder bereitzustellen.

10. Ein integriertes Verankerungsvisualisierungssystem (400), das eine Vorrichtung wie in einem vorangegangenen Anspruch beansprucht aufweist und weiter aufweist:
eine Zuführvorrichtung (200), die ein Zuführrohr (230) mit einem proximalen und distalen Ende und einen Aktuator (220) umfasst;
mit einer Vielzahl von Verankerungsvorrichtungen (12; 112) innerhalb der Zuführvorrichtung (200) angeordnet; wobei
die Zuführvorrichtung (200) ein Gehäuse (205) und einen an dem proximalen Ende angeordneten Handgriff (210) umfasst; und
wobei das integrierte Verankerungsvisualisierungssystem (400) weiter einen Bildschirm (420) zum Anzeigen von Bildern aufweist.

## Revendications

1. Appareil de visualisation chirurgicale (10 ; 100) comprenant :
un dispositif de fixation (12 ; 112) ayant une surface supérieure (14 ; 114) et une surface de pénétration tissulaire (16 ; 116) ;
un dispositif d'imagerie (20, 120) logé dans le dispositif de fixation (12 ; 112), le dispositif d'imagerie comprenant une caméra (22 ; 122) comportant au moins un objectif dirigé vers l'extérieur depuis la surface supérieure (14 ; 114) du dispositif de fixation (12 ; 112) de telle sorte que lorsque le dispositif de fixation (12 ; 112) est inséré dans un tissu, l'au moins un objectif est orienté loin du tissu ;
dans lequel le dispositif de fixation (12 ; 112) est bioabsorbable.

2. Appareil de visualisation chirurgicale (10 ; 100) selon la revendication 1, comprenant en outre une source de lumière.

3. Appareil de visualisation chirurgicale (10 ; 100) selon la revendication 2, dans lequel la source de lumière comporte des diodes électroluminescentes.

4. Appareil de visualisation chirurgicale (10 ; 100) selon une quelconque revendication précédente, comprenant en outre un filtre optique.

5. Appareil de visualisation chirurgicale (10 ; 100) selon une quelconque revendication précédente, dans lequel le dispositif de fixation (12 ; 112) est choisi dans le groupe constitué de rivets, de fléchettes, d'ancres, d'implants barbelés, d'implants serrés, d'attaches et de combinaisons de ceux-ci.

6. Appareil de visualisation chirurgicale (10 ; 100) selon une quelconque revendication précédente, dans lequel au moins une partie de la caméra (22 ; 122) est absorbable.

7. Appareil de visualisation chirurgicale (10 ; 100) selon une quelconque revendication précédente, dans lequel la caméra (22 ; 122) comporte un matériau choisi dans le groupe constitué des verres, des céramiques, des polymères, des métaux et des combinaisons de ceux-ci.

8. Appareil de visualisation chirurgicale (10 ; 100) selon une quelconque revendication précédente, dans lequel la caméra (22 ; 122) est sans fil et comporte des dispositifs choisis dans le groupe constitué des composants à base de fibres optiques, CMOS, CCD, CID, NIR, Raman, fluorescence, des composants à base numérique, des composants à base optique et des variations de ceux-ci.

9. Appareil de visualisation chirurgicale (10 ; 100) selon une quelconque revendication précédente, dans lequel l'au moins un objectif peut pivoter à l'intérieur du dispositif de fixation (12 ; 112) pour capturer des images du tissu afin de fournir des champs de vision préférés.

10. Système de visualisation à fixation intégrée (400), comprenant un appareil tel que revendiqué dans une quelconque revendication précédente, et comprenant en outre :
un dispositif de délivrance (200) comportant un tube de délivrance (230) ayant une extrémité proximale et distale et un actionneur (220) ;
avec une pluralité de dispositifs de fixation (12 ; 112) disposés à l'intérieur du dispositif de délivrance (200) ;
dans lequel :
le dispositif de délivrance (200) comporte un logement (205) et une poignée (210) placée sur l'extrémité proximale ; et
le système de visualisation à fixation intégrée (400) comprend en outre un moniteur (420) pour afficher des images.
